## Europäisches Patentamt
## European Patent Office
## Office européen des brevets

(19)

(11) Numéro de publication : **0 270 553 B1**

(12)
# FASCICULE DE BREVET EUROPEEN

(45) Date de publication du fascicule du brevet :
**21.08.91 Bulletin 91/34**

(51) Int. Cl.⁵ : **A61F 7/02, A61F 7/10**

(21) Numéro de dépôt : **87901331.6**

(22) Date de dépôt : **09.03.87**

(86) Numéro de dépôt international :
**PCT/CH87/00029**

(87) Numéro de publication internationale :
**WO 87/06825 19.11.87 Gazette 87/25**

(54) **DISPOSITIF THERAPEUTIQUE COMPORTANT UNE MASSE D'UN MATERIAU THERMIQUEMENT ACTIF.**

(30) Priorité : **16.05.86 CH 2000/86**

(43) Date de publication de la demande :
**15.06.88 Bulletin 88/24**

(45) Mention de la délivrance du brevet :
**21.08.91 Bulletin 91/34**

(84) Etats contractants désignés :
**AT BE CH DE FR GB IT LI LU NL SE**

(56) Documents cités :
**WO-A-86/03400**
**FR-A- 1 365 666**
**FR-A- 2 171 135**
**GB-A- 2 160 965**

(73) Titulaire : **TERM-ac S.A.**
**Rte St-Nicolas-de-Flüe, 20**
**CH-1700 Fribourg (CH)**

(72) Inventeur : **NOPPEL, René**
**Ramière 7**
**CH-1028 Préverenges (CH)**
Inventeur : **WUILLEMIN, Kurt**
**/**
**CH-1781 Courgevaux (CH)**

(74) Mandataire : **Rochat, Daniel Jean et al**
**Bovard SA Ingénieurs-Conseils ACP**
**Optingenstrasse 16**
**CH-3000 Bern 25 (CH)**

## Description

On sait que certains traitements thérapeutiques comportent l'application sur les parties du corps à traîter de masses froides ou de masses chaudes. Comme exemple de thérapie froide on peut citer le traîtement des contusions ou des incisions opératoires. On utilise pour cela des coussins froids qui se réchauffent progressivement à partir d'une température d'environ 0°C jusqu'à la température du corps. Les matériaux utilisés, par exemple des mélanges d'eau et de glycol cèdent les frigories qu'ils ont emmagasinés selon un processus à élévation constante, donc d'une façon linéaire. Pour que leur effet refroidisseur dure suffisamment longtemps, il faut donc qu'ils soient fortement refroidis. Cependant, l'application de corps à très basse température sur la peau peut être ressentie de façon désagréable et même entraîner des gelures locales. A cela s'ajoute que le rendement de l'application de ces méthodes au point de vue de l'effet thérapeutique désiré est loin d'être optimal.

Ainsi, la plupart des coussins refroidisseurs connus actuellement sur le marché présentent des inconvénients. Comme indiqué ci-dessus, ils perdent rapidement leur effet, de sorte qu'ils doivent être changés fréquemment, ce qui entraîne des variations de température défavorables pour la thérapie visée et exige du personnel soignant un engagement important. Certains coussins refroidisseurs connus, comme par exemple celui qui est décrit dans le brevet allemand DE 3141191 présentent l'inconvénient que le matériau thermiquement actif n'occupe pas l'ensemble du volume du coussin puisqu'il forme une émulsion avec un émulgateur non actif, qui réduit son efficacité.

On a déja songé pour remédier partiellement à ces inconvénients, et notamment à la variation rapide de la température des coussins froids au contact du corps, à utiliser des cubes de glace qui ont l'avantage de rester à la température de 0°C aussi longtemps qu'ils n'ont pas entièrement fondu. La glace peut en effet absorber du corps avec lequel elle est en contact une quantité de chaleur égale à sa chaleur latente de fusion qui est de 314 KJ/kg. Cependant, en fondant, les blocs de glace mouillent les pièces de vêtement avec lesquels ils sont en contact et d'autre part ils présentent généralement à l'état solide des arêtes vives qui peuvent blesser la peau.

On connait également, notamment par le brevet américain US-A- 4,527,566 des bandages souples équipés de poches susceptibles de recevoir des blocs de glace ou des cellules chaudes mais ce bandage ne remédie que partiellement aux inconvénients susmentionnés.

Le brevet français FR-A- 1,365,666 contient aussi une description d'un conditionnement de masses d'un liquide congelable. Ces masses sont enfermées dans des récipients qui sont eux-mêmes placés dans des emballages de formes et constructions diverses.

Le but de la présente invention est de créer un dispositif thérapeutique simple, susceptible d'être utilisé dans des conditions très différentes les unes des autres, pratique à l'emploi et répondant mieux que les dispositifs connus jusqu'à maintenant aux exigences pratiques et thérapeutiques auquels les dispositifs de ce genre sont soumis.

On connaît des dispositifs thérapeutiques à action thermique qui atteignent partiellement ce but et qui comportent une pluralité de cellules formées chacune d'une masse d'un matériau thermiquement actif et présentant un changement d'état à une température prédéterminée, et d'une enveloppe fermée, étanche, à paroi mince contenant la dite masse.

Selon le brevet français FR-A- 2,171,135, les cellules sont solidaires les unes des autres et forment ensemble un coussin.

Le dispositif selon la présente invention se distingue, sous un premier aspect, des objets connus par le document antérieur précité, en ce que la dite pluralité de cellules forme un ensemble de cellules séparées les unes des autres réparties en au moins deux groupes présentant des températures de changement d'état différentes et en ce que le dispositif comporte en outre un bandage avec une ou plusieurs poches dont les dimensions sont adaptées à celles des cellules, ce bandage étant agencé pour recevoir un choix des dites cellules de manière à permettre l'application d'un effet thérapeutique chaud ou froid selon les cellules utilisées.

Selon un autre aspect, le dispositif selon l'invention se distingue en ce que la dite pluralité de cellules forme un ensemble de cellules séparées les unes des autres, noyées dans un agent caloporteur fluide, non gazeux, ayant une fonction d'échange de chaleur, le dit agent caloporteur et les cellules étant contenus dans un emballage fermé, souple.

On va décrire ci-après à titre d'exemple différentes formes d'exécution de l'objet de l'invention en se référant au dessin annexé, dont :

la fig. 1 est une vue en perspective partiellement coupée de la première forme d'exécution,

la fig. 2 une vue en perspective également coupée montrant une cellule contenant le matériau thermiquement actif,

la fig. 3 une vue en coupe de la seconde forme d'exécution du dispositif thérapeutique, et

la fig. 4 un graphique montrant l'application du dispositif.

Comme on le voit au dessin, le dispositif se compose essentiellement d'un bandage 1 pourvu d'un certain nombre de poches 2 ayant toutes les mêmes dimensions, et d'un ensemble de cellules 3 qui contiennent le matériau thermiquement actif.

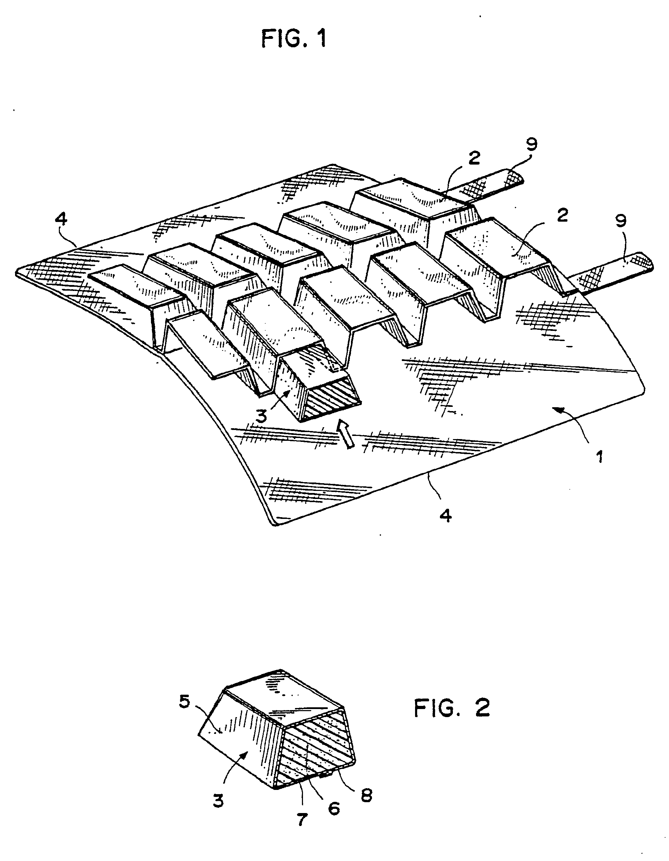

La forme et la constitution du bandage 1 peuvent être très variables. Selon la fig. 1, le bandage est constitué d'un segment de toile de base 4 sur lequel sont fixés d'autres éléments de toile délimitant les poches 2. Ces poches sont disposées en deux rangées. Elles ont approximativement la forme de parallélépipèdes rectangles qui sont adaptés aux dimensions des cellules 3 comme on le verra plus loin. Le bandage peut être entièrement formé d'un tissu ou de feuilles de matière plastique. Dans la forme d'exécution représentée, les bords longitudinaux de la pièce de base 4 peuvent être rabattus sur les deux rangées de poches 2 afin de fermer ces dernières. Bien entendu, à cette pièce de base 4 peuvent être fixées des lanières (non représentées) ou tout autre élément permettant si on le désire, de fixer le bandage sur la partie du corps qui doit être traitée, par exemple autour d'une jambe ou autour d'un bras. Cependant, il est bien entendu que ce bandage peut également être fixé par exemple par des pièces de bande adhésive adhérent directement au corps.

Comme on le voit à l'extrémité gauche de la fig. 1, les poches 2 sont ouvertes sur une de leur face et fermées sur les autres côtés. Des lanières 9 permettent de fixer le bandage à la partie du corps à traiter, par exemple autour d'un bras ou d'une jambe.

Les cellules 3 se composent chacune d'une enveloppe 5 à parois minces qui sera dé préférence en un matériau composite contenant une feuille d'aluminium, ou en une matière plastique étanche à l'air et à l'humidité, et d'une masse de remplissage 6 qui est constituée du matériau thermiquement actif que l'on désire utiliser. Le volume des cellules peut être standardisé. Pour la forme de mise en oeuvre décrite ici, il sera par exemple compris entre 5 à 30 cm³. Bien entendu le volume et la forme des cellules correspondent au volume et à la forme des poches 2. Dans l'exemple donné, il s'agit de blocs à forme trapézoïdale. Les cellules peuvent être formés par mise en forme d'une matière plastique et soudage du couvercle sur la partie ainsi moulée. On peut également constituer les enveloppes 5 à partir de feuilles stratifiées, découpées, pliées et fermées par soudage. Ainsi, à la fig. 2, les parois inférieures des cellules sont formées de deux parties marginales 7 et 8 d'une feuille de départ, soudées l'une sur l'autre par recouvrement.

Le matériau 6 contenu dans les cellules est un matériau qui présente un changement de phases à une température prédéterminée. De préférence, ce changement de phases sera un passage de la phase liquide à la phase solide ou vice versa. La température de transformation pourra être fixée à une valeur comprise entre –15° et +15°C s'il s'agit d'une application de thérapie froide. Au contraire, cette température de transformation pourra être fixée à une valeur comprise entre +35° et +70°C s'il s'agit d'une application de thérapie chaude. On choisira le matériau thermiquement actif de façon que sa chaleur latente de fusion par unité de volume soit aussi élevée que possible. Ainsi par exemple, dans le cas d'une application de thérapie froide, le matériau thermiquement actif peut être un composé chimique du groupe des hydrates salins ou, le cas échéant, l'eau. Dans le cas d'une application de thérapie chaude, on pourra utiliser d'autres matériaux, par exemple des composés à base d'hydrocarbures saturés ou non, ou également des hydrates salins.

Le dispositif décrit permet d'associer des séries de cellules, telles que les cellules 3 en différents matériaux présentant des températures de transformation bien déterminées, avec des bandages de différentes formes et de différentes dimensions aptes à être appliquées à différentes parties du corps humain. L'ensemble donne donc une grande souplesse d'utilisation. Le traitement présente une efficacité améliorée par rapport à l'emploi de matériaux qui se réchauffent progressivement au contact du corps, puisque le bandage appliqué au corps est apte à absorber une grande quantité de chaleur en restant à un palier de température bien déterminé, facile à supporter pour le patient, mais efficace quant à l'effet thermique visé. Le cas échéant on peut même combiner dans un même bandage des cellules ayant deux températures de transformation différentes afin d'obtenir un traitement à deux paliers de température successifs.

Bien entendu, pour améliorer la facilité d'emploi, les différentes cellules 3 pourront être marquées afin de permettre le repérage de leur température de transformation. Elles seront maintenues prêtes à l'emploi, dans des armoires frigorifiques appropriées, pour les cellules froides, dans des enceintes chaudes, pour les autres cellules destinées aux applications à température élevée. L'ensemble des éléments décrits permet une application rationnelle des traitements thérapeutiques par application de chaleur ou de froid, en limitant l'engagement du personnel soignant.

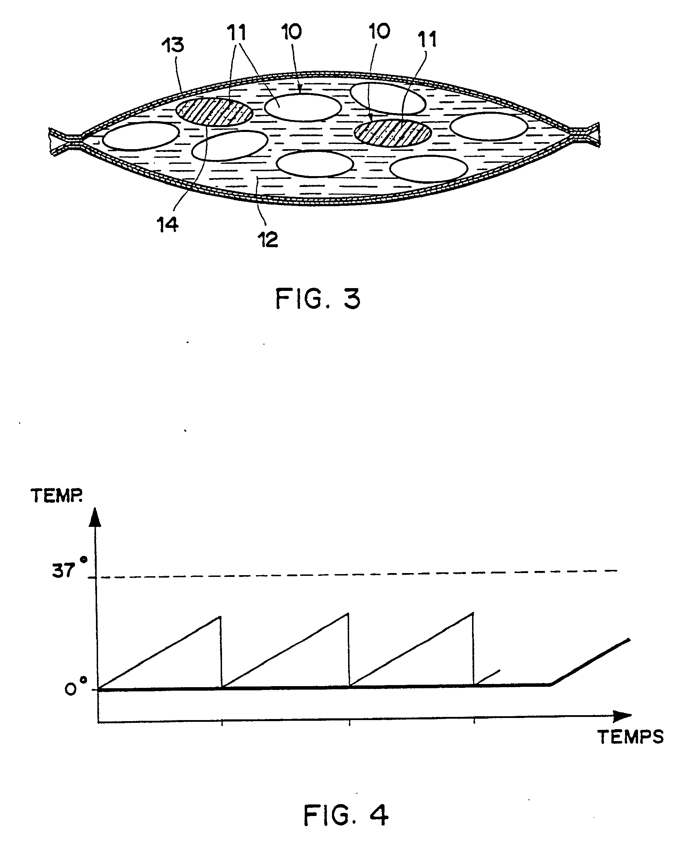

La fig. 3 illustre un autre exemple d'application du principe exposé plus haut : au lieu d'utiliser des cellules telles que les cellules 3 que l'on introduit dans les poches d'un bandage 1, les cellules sont noyées dans une masse d'un corps caloporteur mou, enfermé lui-même à l'intérieur d'une enveloppe fermée étanche, souple et déformable.

Ainsi, la fig. 3 montre des cellules 10 entièrement fermées et contenant chacune une masse prédéterminée d'un matériau thermiquement actif, ce dernier étant désigné par 11. Les cellules 10 sont elles-mêmes noyées dans un gel ou une émulsion désigné par 12 enfermé lui-même à l'intérieur d'une enveloppe 13. Dans ce cas, les doses de matériau thermo-actif contenu dans les cellules seront plus petites que dans le cas de la première application. Leur volume sera par exemple de 0,05 à 1 cm³. Ces doses de matériaux thermiquement actifs seront entourées d'une pellicule de matière plastique formant une enveloppe fermée 14, étanche à parois minces. Pour la réalisation de cette enveloppe, on pourra utiliser les mêmes procédés que pour les enveloppes des cellules 3 représentées à la fig. 2. La forme des cellules sera de préférence différente de celles représentées à la fig. 2. On peut prévoir par exemple des cellules sphériques, à condition que le matériau de l'enveloppe soit extensible élastiquement, de manière à ce que les variations de volume résultant d'un changement de phase soient absorbées. Toutefois on peut également prévoir des formes d'ellipsoïdes par exemple, ou de façon générale des formes s'écartant de la sphère de manière que les variations de volume dues au changement de phases soient compensées par une variation de forme. Dans une application, les cellules peuvent être formées en introduisant le produit thermiquement actif dans un tuyau continu formé du matériau de l'enveloppe. Ensuite, le tuyau est pincé de distance en distance et soudé sur lui-même, puis sectionné, ce qui donne les cellules thermiquement actifs. Outre les produits mentionnés plus haut, les études ont montré que d'autres matériaux conviennent également, comme par exemple des sels hydratés, certains eutectiques, des hydrocarbures. L'eau a déjà été mentionnée. Lorsqu'on utilise l'eau ou une solution saline, il sera avantageux d'y ajouter des adjuvants capables d'éviter la formation de pointes de cristallisation lors de la congélation, et de même pour éviter le phénomène de la surfusion lors du refroidissement.

La masse dans laquelle les cellules de la fig. 3 sont noyées est un fluide caloporteur qui présentera de préférence certaines caractéristiques comme une chaleur spécifique élevée, une bonne fluidité interne de façon à ce que la forme de l'emballage extérieur puisse varier et s'adapter aux parties du corps sur lequel le dispositif s'applique et finalement une bonne stabilité chimique et physique. Certains gels ou émulsions connus répondent particulièrement bien aux exigences. Le cas échéant, l'émulsion peut être constituée de telle façon que la phase dispersée présente elle-même un changement d'état au cours de l'évolution thermique du dispositif. Dans ce cas, la température de transformation de cette phase dispersée sera voisine de la température de transformation du matériau thermiquement actif logé dans les cellules. L'évolution du dispositif permettra d'utiliser la chaleur latente de transformation du gel ou de l'émulsion dans laquelle les sphérules sont noyées.

Enfin, l'enveloppe extérieure sera de préférence un sac en matière plastique souple fermé de façon étanche, par exemple un sac en polyéthylène. Le fait d'utiliser un conditionnement tel que décrit ci-dessus permet d'obtenir un contact optimum entre la matière active et la partie du corps humain à traiter. De façon générale, l'utilisation d'emballages selon la fig. 3 présente de grands avantages. La facilité d'emploi, de stockage, d'entretien des sacs décrits, est très grande. Ils peuvent être lavés facilement. La matière de l'enveloppe est très lisse et souple, ce qui facilite la conformation du sac à la partie du corps sur laquelle elle s'applique. La conformation du gel ou de l'émulsion facilite aussi cette mise en forme. Le sac peut en outre être coloré ou porter des inscriptions. Il peut être garni d'une pellicule d'une matière sensible capable d'indiquer si la température de transformation est atteint.

Finalement, la fig. 4 montre le résultat de tests comparatifs qui ont été exécutés. Alors qu'avec une application usuelle, la température augmente progressivement dès que l'objet est en contact avec le corps, de sorte qu'il faut changer l'application, par exemple tous les quarts d'heure, avec le dispositif selon l'invention, la température de 0°C est maintenue pendant une durée qui est un multiple de celle enregistrée jusqu'à maintenant. On arrive facilement à une durée d'emploi utile de 1 heure si les cellules sont congelées par exemple à 0°C. La masse du fluide caloporteur et les parois des cellules et du sac forment des barrières qui élèvent légèrement la température au contact du corps. Celle-ci est dès lors de l'ordre de quelques degrés, et l'application est ressentie comme nettement plus agréable, tout en gardant le palier de température de longue durée représenté par le diagramme.

## Revendications

1. Dispositif thérapeutique à action thermique comportant une pluralité de cellules (3 ; 10) formées chacune d'une masse (6 ; 11) d'un matériau thermiquement actif et présentant un changement d'état à une température prédéterminée, et d'une enveloppe (5 ; 14) fermée, étanche, à paroi mince, contenant la dite masse, caractérisé en ce que la dite pluralité de cellules forme un ensemble de cellules séparées les unes des autres réparties en au moins deux groupes présentant des températures de changement d'état différentes et en ce que le dispositif comporte en outre un bandage (1) avec une pluralité de poches (2) dont les dimensions sont adaptées à celles des cellules (3), ce bandage étant agencé pour recevoir un choix des dites cellules (3) de manière à permettre l'application d'un effet thérapeutique chaud ou froid selon les cellules utilisées.

2. Dispositif selon la revendication 1, caractérisé en ce que le changement d'état du matériau thermiquement actif de chaque cellule est un passage de la phase solide à la phase liquide ou vice versa, la température de transformation étant comprise entre −15° et +15°C dans le cas d'une application thérapeutique froide et entre +35° et +70°C dans le cas d'une application thérapeutique chaude.

3. Dispositif selon la revendication 2, caractérisé en ce que des groupes de cellules (3) avec des températures de changement d'état différentes sont prévus de manière à permettre des traitements à plusieurs paliers de température successifs.

4. Dispositif selon l'une quelconque des revendications précédentes, caractérisé en ce que l'enveloppe (5 ; 14) des cellules comporte une feuille stratifiée en un matériau plastique, étanche à l'air et à l'humidité.

5. Dispositif selon l'une quelconque des revendications précédentes, caractérisé en ce que les cellules (3 ; 10) présentent une forme différente de la sphère de manière a pouvoir absorber des variations de volume par des variations de forme.

6. Dispositif selon l'une quelconque des revendications 1 à 4, caractérisé en ce que l'enveloppe des cellules est conformée de manière à permettre une variation de volume lors du changement d'état du matériau thermiquement actif.

7. Dispositif selon la revendication 6, caractérisé en ce que le matériau de l'enveloppe des cellules est un matériau susceptible de s'étendre élastiquement.

8. Dispositif selon la revendication 1, caractérisé en ce que le matériau thermiquement actif des cellules est un sel, un sel hydraté, un eutectique, un hydrocarbure ou l'eau.

9. Dispositif selon la revendication 1, caractérisé en ce que le bandage (1) est en une pièce d'un matériau mince et souple, dont une partie (4) est rabattable sur l'ouverture des poches (2) de manière à éviter le contact direct de l'enveloppe des cellules (3) avec la partie du corps soumise au traitement, sans entraver la transmission de chaleur entre cette partie du corps et les cellules.

10. Dispositif thérapeutique a action thermique comportant une pluralité de cellules (10) formées chacune d'une masse (11) d'un matériau thermiquement actif et présentant un changement d'état à une température prédéterminée et d'une enveloppe (14) fermée, étanche, à paroi mince, contenant la dite masse, caractérisé en ce que la dite pluralité de cellules forme un ensemble de cellules séparées les unes des autres, noyées dans un agent caloporteur (12) fluide, non gazeux, ayant une fonction d'échange de chaleur, le dit agent caloporteur (12) et les cellules (10) étant contenus dans un emballage (13) fermé, souple.

11. Dispositif thérapeutique selon la revendication 10, caractérisé en ce que l'agent caloporteur (12) est un gel, une émulsion, ou un autre fluide non gazeux.

12. Dispositif selon la revendication 11, caractérisé en ce que l'emballage (13) est constitué d'une feuille de polyéthylène, de nylon, de PVC, ou de polypropylène.

13. Dispositif selon la revendication 10, caractérisé en ce que l'emballage (13) est constitué d'une feuille composite ayant une constitution apte à garantir l'étanchéité et la souplesse du dispositif, ce dernier étant utilisé comme coussin chaud ou froid.

**Patentansprüche**

1. Therapeutische Vorrichtung zur Wärmebehandlung, wobei vorhanden sind eine Mehrzahl von Zellen (3, 10), die jeweils aus einer Masse (6, 11) eines thermisch aktiven Materials gebildet sind und bei einer vorbestimmten Temperatur eine Zustandsänderung aufweisen, und eine geschlossene, undurchlässige, dünnwandige Umhüllung (5, 14), welche die erwähnte Masse enthält, dadurch gekennzeichnet, dass die erwähnte Mehrzahl von Zellen eine Anordnung von voneinander getrennten Zellen sind, die in wenigstens zwei Gruppen angeordnet sind, welche unterschiedliche Zustandsänderungs-Temperaturen aufweisen, und dass die Vorrichtung ausserdem eine Bandage (1) mit einer Mehrzahl von Taschen (2) umfasst, deren Abmessungen an diejenigen der Zellen (3) angepasst sind, welche Bandage dazu bestimmt ist, eine Auswahl der erwähnten Zellen (3) derart aufzunehmen, dass je nach verwendeten Zellen eine Behandlung mit kaltem oder warmem therapeutischem Effekt ermöglicht wird.

2. Vorrichtung nach Anspruch 1, dadurch gekennzeichnet, dass der Wechsel des Zustandes des thermisch aktiven Materials jeder Zelle ein Uebergang von der festen Phase in die flüssige Phase oder umgekehrt ist, wobei die Uebergangstemperatur im Falle einer kalten therapeutischen Behandlung zwischen −15° und +15°C liegt und im Falle einer warmen therapeutischen Behandlung zwischen +35° und +70° C.

3. Vorrichtung nach Anspruch 2, dadurch gekennzeichnet, dass die Gruppen von Zellen (3) mit verschiedenen Zustandsänderungs-Temperaturen vorgesehen sind, um mehrstufige Behandlungen mit aufeinanderfolgenden Temperaturen zu ermöglichen werden.

4. Vorrichtung nach einem der vorstehenden Ansprüche, dadurch gekennzeichnet, dass die Umhüllung (5, 14) der Zellen eine geschichtete Folie aus Kunststoffmaterial aufweist, die luft- und feuchtigkeitsdicht ist.

5. Vorrichtung nach einem der vorstehenden Ansprüche, dadurch gekennzeichnet, dass die Zellen (3, 10) eine vom sphärischen verschiedene Form haben, um Volumenänderungen durch Formänderungen absorbieren zu können.

6. Vorrichtung nach einem der Ansprüche 1 bis 4, dadurch gekennzeichnet, dass die Umhüllung der Zellen so ausgebildet ist, dass eine Volumenänderung während des Zustandswechsels des thermisch aktiven Materials möglich ist.

7. Vorrichtung nach Anspruch 6, dadurch gekennzeichnet, dass das Material der Umhüllung der Zellen ein Material ist, welches in der Lage ist, sich elastisch auszudehnen.

8. Vorrichtung nach Anspruch 1, dadurch gekennzeichnet, dass das thermisch aktive Material der Zellen ein Salz ist, ein hydriertes Salz, ein Eutektikum, eine Kohlenwasserstoffverbindung oder Wasser.

9. Vorrichtung nach Anspruch 1, dadurch gekennzeichnet, dass die Bandage (1) aus einem Stück eines dünnen und weichen Materials besteht, deren einer Bereich (4) über die Oeffnungen der Taschen (2) umklappbar ist, um einen direkten Kontakt der Umhüllung der Zellen (3) mit dem darunterliegenden zu behandelnden Körperteil zu vermeiden, ohne die Uebertragung der Wärme zwischen diesem Körperteil und den Zellen zu behindern.

10. Therapeutische Vorrichtung zur Wärmebehandlung, wobei vorhanden ist eine Mehrzahl von Zellen (10), die jeweils aus einer Masse (11) von thermisch aktivem Material bestehen und bei einer vorbestimmten Temperatur einen Zustandswechsel aufweisen, und eine geschlossene, undurchlässige, dünnwandige Umhüllung (14), welche die erwähnte Masse enthält, dadurch gekennzeichnet, dass die erwähnte Mehrzahl von Zellen eine Anordnung von voneinander getrennten Zellen ist, die in einem fluidischen, nicht gasförmigen Wärmeübertragungsmittel (12) eingelassen sind, welches die Funktion der Uebertragung von Wärme hat, wobei das erwähnte Wärmeübertragungsmittel (12) und die Zellen (10) in einer geschlossenen, weichen Verpackung (13) enthalten sind.

11. Therapeutische Vorrichtung nach Anspruch 10, dadurch gekennzeichnet, dass das Wärmeübertragungsmittel (12) ein Gel ist, eine Emulsion oder ein anderes nicht gasförmiges Fluid.

12. Vorrichtung nach Anspruch 11, dadurch gekennzeichnet, dass die Verpackung (13) aus einer Polyethylenfolie, Nylon, PVC oder Propylene besteht.

13. Vorrichtung nach Anspruch 10, dadurch gekennzeichnet, dass die Verpackung (13) aus einer Kompositfolie mit Eigenschaften besteht, die in der Lage sind, die Undurchlässigkeit und die Weichheit der Vorrichtung zu garantieren, wobei diese als warmes oder kaltes Kissen benutzt wird.

## Claims

1. A thermal-action therapeutic device comprising a plurality of cells (3 ; 10) each formed of a mass (6 ; 11) of a thermally active material and presenting a change of state at a predetermined temperature, and of a closed, fluid-tight envelope (5 ; 14) with thin walls containing the said mass, characterized in that the said plurality of cells forms an assembly of cells separated one from the other and divided into at least two groups having different temperatures of change of state and in that the device also comprises a bandage (1) with a plurality of pockets (2) whose dimensions are adapted to those of the cells (3), this bandage being arranged to receive a selection of the said cells (3) in such a way as to allow the application of a hot or cold therapeutic effect, depending on the cells used.

2. Device according to claim 1, characterized in that the change of state of the thermally active material of each cell is a passage from the solid phase to the liquid phase or vice versa, the temperature of transformation being comprised between −15° and +15°C in the case of a cold therapeutic application and between +35° and +70°C in the case of a hot therapeutic application.

3. Device according to claim 2, characterized in that groups of cells (3) with different temperatures of change of state are provided so as to allow several stages of treatment at successive temperatures.

4. Device according to any one of the preceding claims, characterized in that the cell envelope (5 ; 14) comprises a stratified sheet of a plastic material, airtight and moisture-tight.

5. Device according to any one of the preceding claims, characterized in that the cells (3 ; 10) have a form other than a sphere so as to be able to absorb variations in volume by variations in shape.

6. Device according to any one of claims 1 to 4, characterized in that the cell envelope is conformed to as to allow a variation in volume at the time of the change in state of the thermally active material.

7. Device according to claim 6, characterized in that the material of the cell envelope is a material capable of stretching elastically.

8. Device according to claim 1, characterized in that the thermally active material of the cells is a salt, a hydrated salt, a eutectic, a hydrocarbon or water.

9. Device according to claim 1, characterized in that the bandage (1) is in one piece of thin and flexible material, of which one part (4) can be closed over the opening of the pockets (2) so as to prevent direct contact between the cell envelope (3) and the part of the body undergoing treatment, without obstructing the transfer of heat between this part of the body and the cells.

10. Thermal-action therapeutic device comprising a plurality of cells (10) each formed of a mass (11) of a thermally active material and presenting a change of state at a predetermined temperature and of a closed, fluid-tight envelope (14) with thin walls, containing the said mass, characterized in that the said plurality of cells forms an assembly of cells separated one from the other, embedded in a fluid, non-gaseous heat-exchanging agent (12) having a heat-exchange function, the said heat-exchanging agent (12) and the cells (10) being contained in a closed, flexible wrapping (13).

11. Therapeutic device according to claim 10, characterized in that the heat-exchanging agent (12) is a gel, an emulsion or another non-gaseous fluid.

12. Device according to claim 11, characterized in that the wrapping (13) is made up of a sheet of polyethylene, of nylon, of PVC or of polypropylene.

13. Device according to claim 10, characterized in that the wrapping (13) is made up of a composite sheet having a constitution capable of guaranteeing the fluid-tightness and the flexibility of the device, the latter being used as a hot or cold pad.

# FIG. 1

# FIG. 2

8

FIG. 3

FIG. 4